Europäisches Patentamt

European Patent Office

Office européen des brevets

(19)

(·) Veröffentlichungsnummer: **0 281 091**
**A2**

## EUROPÄISCHE PATENTANMELDUNG

(21) Anmeldenummer: 88103142.1

(22) Anmeldetag: 02.03.88

(51) Int. Cl.⁴ **A23K 1/00** , A61K 35/74 , C12N 1/20 , //(C12N1/20, C12R1:12)

Der (Die) Mikroorganismus (Mikroorganismen) ist (sind) bei American Type Culture Collection unter der (den) Nummer(n) 21551 hinterlegt worden.

(30) Priorität: 06.03.87 DE 3707160

(43) Veröffentlichungstag der Anmeldung: 07.09.88 Patentblatt 88/36

(84) Benannte Vertragsstaaten: BE CH DE ES FR GB IT LI NL SE

(71) Anmelder: HOECHST AKTIENGESELLSCHAFT Postfach 80 03 20 D-6230 Frankfurt am Main 80(DE)

(72) Erfinder: Seeger, Karl, Dr. Schwalbenweg 9 D-6238 Hofheim am Taunus(DE) Erfinder: Sambeth, Walther, Dr. Milcheshohl 10 D-6240 Königstein/Taunus(DE) Erfinder: Bauer, Fritz, Dr. Oranienstrasse 46 D-6232 Bad Soden am Taunus(DE)

(54) Bacillus polymyxa enthaltende Mittel zur Verabreichung an Tiere.

(57) Gegenstand der Erfindung sind Mittel zur Verabreichung an Tiere, die ein Bakterienstamm von Bacillus polymyxa enthalten sowie die Verwendung von B. polymyxa zur Wachstumsförderung von Tieren.

EP 0 281 091 A2

## Bacillus polymyxa enthaltende Mittel zur Verabreichung an Tiere.

Bacillus polymyxa, seine Kultivierung und Isolierung ist aus F.H. Grau, P.W. Wilson, J. Bacteriol.83 (1962) 490 bekannt. Weiterhin wird die Kultivierung von B. polymyxa und dessen Verwendung zum Aufschluß von Pflanzenmaterial in US PS 3,812,012 beschrieben. Dort wird auch ein spezieller Stamm genannt, der bei der Hinterlegungsstelle American Type Culture Collection (ATCC) unter der Reg. Nr. 21551 aufbewahrt wird und öffentlich zugänglich ist.

Es wurde nun überraschenderweise gefunden, daß nach Verabreichung von Bacillus polymyxa an Tiere eine deutliche Verbesserung der Gewichtszunahme eintritt.

Gegenstand der Erfindung sind daher Mittel zur Verabreichung an Tiere, dadurch gekennzeichnet, daß sie ein Stamm von Bacillus polymyxa enthalten.

Bacillus polymyxa kann dem Tierfutter oder dem Trinkwasser der Tiere, insbesondere dem Tierfutter, zugefügt werden oder in Form von Tabletten, Boli etc. verabreicht werden. Es können natürliche Isolate oder bereits hinterlegte Stämme sowie deren Mutanten oder Varianten, insbesondere der unter der ATCC Reg.Nr. 21551 hinterlegte Bakterienstamm oder dessen Mutanten oder Varianten, erfindungsgemäß eingesetzt werden.

Der Zusatz von B. polymyxa zur Tiernahrung hilft, die verabreichten Futterstoffe besser zu nutzen, also bei gleichbleibender Futtermenge eine bessere Mastleistung zu erzielen. Weiterhin wird eine Stabilisierung der Darmflora beobachtet. Diese Wirkung ist vor allem in Streßsituationen der Tiere z.B. bei Umstallung, Futterwechsel, Absetzen etc. von Bedeutung.

Die erfindungsgemäßen Mittel können daher auch im Falle von Störungen im Bereich des Magen-Darmtraktes durch bakterielle Infektionen, z.B. zur Verhinderung oder Bekämpfung von Durchfallerkrankungen eingesetzt werden.

Diese Mehrfachwirkung von B. polymyxa führt dazu, daß sich die Tiere besser entwickeln, und eine geringere Zahl von Erkrankungen beobachtet wird. Insbesondere ist dieser Effekt ausgeprägt bei solchen Tieren, die ein an Pektinen reichhaltiges Futter oder Futter mit niedrigem Eiweißgehalt erhalten.

Gegenstand der Erfindung ist daher auch ein Verfahren zur Verabreichung von B. polymyxa an Tiere, insbesondere im Tierfutter oder im Trinkwasser. B. polymyxa kann aber auch als Suspension oder in Form von Tabletten, Dragees oder mit Hilfe von Systemen, die eine verzögerte Freisetzung gewährleisten, insbesondere in einem Bolus, verabreicht werden. Zur Formulierung werden jeweils die üblichen Hilfsstoffe zugesetzt.

Der Gehalt an B. polymyxa im Tierfutter kann innerhalb weiter Grenzen variieren. Er liegt im allgemeinen zwischen $10^4$ und $10^{12}$ Sporen/kg Futter, insbesondere zwischen $10^6$ bis $10^{10}$ Sporen/kg.

Der Gehalt im Trinkwasser kann variieren zwischen $10^4$ und $10^{12}$ Sporen/l.

B. polymyxa kann allgemein an Nutztiere z.B. an Wiederkäuer wie Rinder, Schafe, Ziegen; an Schweine; Kaninchen; Pferde; Fische; an Geflügel wie Hühner, Puten, Gänse, Enten oder Wachteln, insbesondere an Schweine, Hühner und Wiederkäuer verabreicht werden.

Die B. polymyxa-Konzentrationen der erfindungsgemäßen Mittel sind auf Futter-oder Trinkwasserzubereitungen ad libitum bezogen, d.h. zur freien Futter-oder Trinkwasseraufnahme während einer praxisüblichen Mast-oder Aufzuchtperiode, insbesondere bei Futter-und Haltungsänderungen. Aufgrund besonderer durch die Praxis bedingter Faktoren kann es sich aber ergeben, daß der Fachmann diese Anwendungskonzentrationen nach oben anpassen muß, falls die Tiere mit verschiedenen Futter-oder Wasservorräten versorgt werden müssen. Dann enthält aber nur ein Teil der Futter-oder Wasservorräte die erfindungsgemäßen Mittel.

Als Träger für die erfindungsgemäßen Mittel eignen·sich alle in der Mischfutterindustrie üblichen Futterformulierungen.

Sie können Vitaminkonzentrate, Mineralkonzentrate oder sonstige Wirkstoffe und Futterzusätze in beliebiger Konzentration enthalten. Sowohl herkömmliche trockene, mehlige oder pelletierte Futtermittel als auch flüssige Suspensionsfutter unter Einschluß von Futtermitteln, wie Destillationsschlempen und Milchnebenprodukten, können bei den erfindungsgemäßen Mitteln verwendet werden.

Für die Herstellung des erfindungsgemäßen Futters setzt man gewöhnlich zuerst ein konzentriertes Vorgemisch an, das B. polymyxa in hoher Konzentration, beispielsweise von 0,2 bis 75 %, enthält. Zu diesem Zweck werden die Mittel mit physiologisch unbedenklichen Trägern, wie Propylenglykolen, Polyethylenglykolen, inerten Ölen, wie Pflanzenölen, hochraffinierten Mineralölen, Äthanol, Wasser, wässrigen Alkoholen, entweder dispergiert oder in inerte Trägerstoffe eingemischt, wie Vermikulit, Diatomeenerde, Attapulgit, Kalziumkarbonat, Bolus alba. Ebenso eignen sich hierfür organische Trägermaterialien, wie Weizenkleie, Maisschrot, Sojabohnenmehl, Luzernmehl, Reishülsen oder gemahlene Maiskolben und beliebig andere organische Trägerstoffe aus pflanzlichen Produkten.

Die erfindungsgemäßen Mittel lassen sich ferner auch zusammen mit dem Trinkwasser verabreichen. Ihre Einarbeitung in das Trinkwasser wird durch Zusatz einer in Wasser suspendierbaren Form der jeweiligen Mittel zu dem Trinkwasser in geeigneter Menge erreicht. Die Herstellung solcher Zubereitungen erfolgt im allgemeinen durch Auswahl einer wasserunlöslichen Form, beispielsweise einer Suspension. Zur Herstellung der Zubereitungen verwendet man physiologisch unbedenkliche Hilfsmittel, durch die die erfindungsgemäßen Mittel in Wasser über längere Zeit in Suspensionen gehalten werden. Hilfsmittel, die sich hierfür eignen sind Quellmittel, wie Alginate, Gelatine, Carboxymethylcellulose oder Polyvinylpyrrolidon. Die erfindungsgemäßen Mittel können aber auch mit verschiedenen oberflächenaktiven Verbindungen suspendiert werden, wie beispielsweise mit Hilfe von Lecithin, Naphthalinsulfonaten, Alkylbenzolsulfonaten, alkylphenol-Polyethylenoxid-Addukten oder Polyoxyethylensorbitanestern. Üblicherweise stellt man zunächst eine konzentrierte Suspension oder auch eine trockene Formulierung aus den erfindungsgemäßen Mitteln und den Suspendierungsmitteln in bestimmten Mischungsverhältnissen her und verdünnt sie dann mit dem Trinkwasser auf die gewünschte Anwendungskonzentration, oder die Vormischungen werden in geeigneten Konzentrationen mit den in der Praxis üblichen Futtermitteln gemischt. Das so aufbereitete Trinkwasser bzw. Futter wird dann dem Tier entweder zunächst ad libitum oder restriktiv angeboten.

Das erfindungsgemäße Verfahren läßt sich auch auf andere Verfahren zur Behandlung und Fütterung von Tieren erweitern. So können beispielsweise die erfindungsgemäßen Zubereitungen mit anderen Wirkstoffen kombiniert werden, wie z.B. mit anderen wachstumsfördernden Mitteln oder Antiparasitika, die einerseits synthetische Mittel oder andererseits Fermentationsprodukte im weitesten Sinn darstellen.

Die Erfindung wird durch nachstehende Beispiele erläutert.

A. Beispiele für Futterzusammensetzungen

|  | Ferkelaufzucht futter (% Anteile) | Masthähnchen- futter (% Anteile) |
|---|---|---|
| Fischmehl | 4,00 | 6,00 |
| Leinkuchenmehl | 5,50 | - |
| Futterhefe | 2,00 | 2,00 |
| Sojaschrot | 16,00 | 24,00 |
| Luzernegrünmehl | 4,00 | 1,00 |
| Gerste | 20,00 | |
| Hafer | 6,00 | |
| Mais | 25,03 | 44,89 |
| Weizen | 10,00 | 6,35 |
| Weizenkleie | 5,00 | - |
| Kohlens. Futterkalk | 1,25 | 0,96 |
| Phosphors. Futterkalk | 0,56 | 1,40 |
| Viehsalz | 0,26 | 0,09 |
| Spurenelementmischung | 0,20 | 0,04 |
| Rindertalg | - | 4,70 |
| Weizennachmehl | - | 8,50 |
| DL-Methionin | - | 0,07 |
| Vormischung* mit | | |
| B. polymyxa | 0,20 | 0,20 |
| | 100,00 | 100,00 |

* B. polymyxa-Konzentration in der Vormischung entspricht $10^6$ - $10^{10}$ B. polymyxa-Sporen/kg Fertigfutter

## Schweinefutter

| Komponenten | normales Anfangsmast-futter (% Anteile) | pektin-haltiges Mastfutter (% Anteile) | proteinarmes Anfangsmast-futter (% Anteile) |
|---|---|---|---|
| Sojaschrot | 26,9 | 26,9 | 19,7 |
| Gerste | 30,0 | 15,0 | 30,0 |
| Mais | 30,0 | 30,0 | 30,0 |
| Tapioka | 5,0 | 9,6 | 13,25 |
| Ca-Phosphat | 1,6 | 1,8 | 1,85 |
| $CaCO_3$ | 6,3 | 1,5 | 5,0 |
| Rübenschnitzel | - | 15,0 | - |
| Spurenelemente - Prämix für Schweine | 0,2 | 0,2 | 0,2 |
| Vitamin-Prämix für Schweine | X | X | X |
| | 100,0 | 100,0 | 100,0 |

B. Tierversuche

Beispiel I

Hähnchenmastversuch

Es wurden Versuchsgruppen von je 4 x 8 Tieren in Batterien ausgewählt. Eine Gruppe diente als Kontrolle. Das Futter enthielt 22 % Rohprotein und 13,2 kJ/kg umsetzbare Energie.. Die Zusammensetzung des Futters erfolgte ad libitum. Nach 3 Versuchswochen wurden folgende Ergebnisse erzielt.

Tabelle I

| B. polymyxa Konzentration (Sporen/kg Futter) | verbesserte Wachstumszunahme (%) | verbesserte Futterverwertung (%) |
|---|---|---|
| $10^6$ | 4,0 | 2,6 |
| $10^7$ | 3,5 | 2,0 |
| $10^8$ | 6,5 | 2,9 |

Die Prozentsätze der verbesserten Wachstumszunahme und verbesserten Futterverwertung beziehen sich auf die Ergebnisse bei den Kontrolltieren ohne B. polymyxa-Zusatz.

**Beispiel II**

Schweinefütterungsversuch

Es wurden Versuchsgruppen von je 20 Tieren gebildet. Eine Gruppe diente als Kontrolle. Das durchschnittliche Anfangsgewicht betrug 16,6 kg. Die Tiere waren mehreren Streßfaktoren ausgesetzt: man verwendete ein proteinarmes Futter (s. unter A); es wurden Tiere aus verschiedenen Herkünften eingesetzt, die zu verschiedenen Terminen eingestallt wurden. Die Fütterung erfolgte restriktiv. Die Ergebnisse sind in folgender Tabelle dargestellt.

Tabelle II

| B. polymyxa Konzentration (Sporen/kg Futter) | Abnahme der kümmernden Tiere (%) | verbesserte Gewichtszunahme (%) | Abnahme Durchfallerscheinungen (%) |
|---|---|---|---|
| $10^8$ | -55 | +20 | -35 |
| $10^{10}$ | -36 | +45 | -46 |

Die Prozentwerte beziehen sich auf die jeweiligen Ergebnisse bei den Kontrolltieren ohne B. polymyxa-Zusatz.

**Ansprüche**

1. Mittel zur Verabreichung an Tiere, dadurch gekennzeichnet, daß sie eine wirksame Menge eines Stammes von Bacillus polymyxa enthalten.

2. Mittel gemäß Anspruch 1, dadurch gekennzeichnet, daß sie als Futtermittel verabreicht werden.

3. Mittel gemäß Anspruch 1, dadurch gekennzeichnet, daß sie als Trinkwasser verabreicht werden.

4. Mittel gemäß Anspruch 1, dadurch gekennzeichnet, daß sie als Suspension oder in Form von Tabletten, Dragees oder Boli verabreicht werden.

5. Mittel gemäß Ansprüchen 1 bis 4, dadurch gekennzeichnet, daß sie zur Bekämpfung oder Verhinderung von Darmentzündungen oder Durchfallerkrankungen eingesetzt werden.

6. Mittel gemäß Ansprüchen 1 bis 4, dadurch gekennzeichnet, daß sie als Wachstumsförderer eingesetzt werden.

7. Mittel gemäß einem oder mehreren der Ansprüche 1 bis 6, dadurch gekennzeichnet, daß sie an Geflügel, Wiederkäuer, Schweine, Pferde, Fische oder Kaninchen verabreicht werden.

8. Mittel gemäß einem oder mehreren der Ansprüche 1, 2, 5, 6 oder 7, dadurch gekennzeichnet, daß der Gehalt an B. polymyxa $10^4$ bis $10^{12}$ Sporen pro kg Tierfutter beträgt.

9. Mittel gemäß einem oder mehreren der Ansprüche 1, 2, 5, 6 oder 7, dadurch gekennzeichnet, daß der Gehalt an B. polymyxa $10^6$ bis $10^{10}$ Sporen pro kg Tierfutter beträgt.

10. Verwendung von Bacillus polymyxa zur Wachstumsförderung bei Tieren.

11. Verfahren zur Wachstumsförderung bei Tieren, dadurch gekennzeichnet, daß man ihnen eine wirksame Menge eines Mittels gemäß einem oder mehreren der Ansprüche 1 bis 7 zuführt.

12. Verfahren zur Herstellung eines Mittels zur Verabreichung an Tiere enthaltend Bacillus polymyxa gemäß einem oder mehreren der Ansprüche 1 bis 9, dadurch gekennzeichnet, daß man Bacillus polymxa mit üblichen Zusatzstoffen in eine geeignete Zubereitungsform bringt.